# EUROPEAN PATENT SPECIFICATION

(11) **EP 4 013 397 B1**
(45) Date of publication and mention of the grant of the patent: **21.10.2026**
(21) Application number: 20764793.4
(22) Date of filing: 12.08.2020
(51) Int. Cl.: A61K 31/05, A61P 1/16, A61P 3/06, A61P 3/10, A61K 45/06

(54) **CANNABIGEROL ALONE OR IN COMBINATION WITH CBD FOR TREATING NON-ALCOHOLIC FATTY LIVER DISEASE, DYSLIPIDEMIA, AND TYPE 2 DIABETES**
CANNABIZEROL ALLEIN ODER IN KOMBINATION MIT CBD ZUR BEHANDLUNG VON NICHTALKOHOLISCHER FETTLEBERERKRANKUNG, DYSLIPIDÄMIE UND TYP-2-DIABETES
CANNABIGÉROL SEUL OU EN COMBINAISON AVEC CBD POUR TRAITER LA STÉATOSE HÉPATIQUE NON ALCOOLIQUE, LA DYSLIPIDÉMIE ET LE DIABÈTE DE TYPE 2

(30) Priority: 13.08.2019 US 201962886015 P
(43) Date of publication of application: 22.06.2022
(73) Proprietor: YISSUM RESEARCH DEVELOPMENT COMPANY OF THE HEBREW UNIVERSITY OF JERUSALEM LTD, 9139002 Jerusalem (IL)
(72) Inventor: TAM, Joseph, 9692850 Jerusalem (IL)
(74) Representative: Fleuchaus & Gallo Partnerschaft mbB
(86) International application number: PCT/IL2020/050885
(87) International publication number: WO 2021/028917

(56) References cited:
- WO-A1-2019/077611
- GB-A- 2 450 493
- SILVESTRI CRISTOFORO ET AL: "Two non-psychoactive cannabinoids reduce intracellular lipid levels and inhibit hepatosteatosis", JOURNAL OF HEPATOLOGY, ELSEVIER, AMSTERDAM, NL, vol. 62, no. 6, 13 January 2015 (2015-01-13), pages 1382 - 1390, XP029173064, ISSN: 0168-8278, DOI: 10.1016/J.JHEP.2015.01.001

## Description

### TECHNOLOGICAL FIELD

The present invention generally relates to phytocannabinoids, particularly cannabigerol and cannabidiol, and their uses in medicine, especially in preventing or treating non-alcoholic liver disease, dyslipidemia and type-2 diabetes.

### BACKGROUND

Obesity is a chronic disease that is now reaching epidemic proportions, with more than one-third of U.S. adults that are considered obese. It has been described as a catalyst for cardiovascular disease, type 2 diabetes (T2D) and non-alcoholic fatty liver disease (NAFLD). The latter, a major cause of morbidity and mortality in Western societies, describes a spectrum of liver conditions ranging from ectopic accumulation of fat in the liver (hepatic steatosis) to non-alcoholic steatohepatitis (NASH), which can be complicated by fibrosis, cirrhosis and hepatocellular carcinoma (HCC). In response to nutritional and hormonal signals, the liver regulates several features of lipid metabolism including free fatty acid (FFA) β-oxidation, *de novo* lipogenesis and lipoprotein uptake and secretion. Hepatic steatosis is a result of increased hepatic lipogenesis combined with decreased FFA β-oxidation as well as increased transport of FFAs to the liver from extra-hepatic (primarily adipose) tissues.

Several lines of evidence suggest that NAFLD promotes T2D. While NAFLD is present in 20-30% of the general population, it reaches the impressive prevalence of 50-75% of patients affected by T2D. Conversely, insulin resistance, which occurs in 66-83% of patients with NAFLD, increases the FFAs flux from adipocytes to the liver, promotes hepatic lipid accumulation and liver injury. Once T2D is fully developed, it further contributes not only to the development of steatosis, but also to NASH, fibrosis, cirrhosis and possibly HCC [1]. To date, NAFLD is considered as a promising, new predictive marker for T2D, with potential therapeutic implications. Therefore, diagnostic and early therapeutic interventions are needed for treating NAFLD patients at risk for developing T2D. While several metabolic factors have been linked to the development of NAFLD, more efforts to achieve a better understanding of the molecular mechanisms involved in lipid metabolism are crucial to the development of novel therapeutic strategies.

Recent findings have revealed a significant role of the endocannabinoid (eCB) system in the pathogenesis of NAFLD via the regulation of both CB1 and CB2 receptors [2]. eCBs are endogenous lipid ligands that interact with the same cannabinoid receptors, which also recognize Δ⁹-tetrahydrocannabinol (THC), the psychoactive component of marijuana and mediate its biological effects. By activating CB1 receptors, eCBs increase appetite (the 'munchies') and lipogenesis in adipose tissue and liver and induce insulin resistance and dyslipidemia. These effects suggest that an overactive eCB/CB 1 receptor system contributes to the development of visceral obesity, T2D and their complications [3]. While CB1 receptors are present at very high levels in the brain, they are also present at much lower, yet functionally relevant, levels in many peripheral tissues, including the liver. In fact, obesity-induced hepatic steatosis depends on the activation of peripheral, including hepatic CB1 receptors. Briefly, the hepatic rate of *de novo* lipogenesis and related gene expression are increased by CB1 agonists and decreased by either globally acting or peripherally restricted CB1 antagonists in rodents [4-11].

Besides eCBs, Cannabis per se, via its non-psychoactive phytocannabinoids, Δ⁹-tetrahydrocannabivarin (THCV) and cannabidiol (CBD), has been shown to modulate fatty acid accumulation in the liver [12]. In fact, CBD inhibits weight gain in rats on high-fat diets and the development of alcohol-induced hepatosteatosis in mice **[13,14],** whereas THCV improves insulin sensitivity and decreases triglyceride (TG) accumulation within the livers of obese mice [15]. These data indicate that phytocannabinoids are able to potentially elicit positive metabolic effects under fatty conditions (such as an unbalanced diet).

Silvestri C. et al. discloses in the article "two non-psychoactive cannabinoids reduce intracellular lipid levels and inhibit hepatosteatosis" (Journal of hepatology, 2015, 62(6), 1382-1380) that THCV and CBD are suggestive to be used as new therapeutic agents for the pharmacological treatment of obesity - and metabolic syndrome-related NAFLD/hepatosteatosis.

WO2019077611A1 discloses a composition based on CBD and chitosan for use in reducing blood cholesterol, blood low-density lipoprotein and blood triglycerides levels.

GB2450493A discloses CBG for use in treatment of diseases benefitting from agonism of CB1 and CB2 cannabinoid receptors.

### REFERENCES

[1] Smith, B.W. and L.A. Adams, Nonalcoholic fatty liver disease and diabetes mellitus: pathogenesis and treatment. Nat Rev Endocrinol, 2011. 7(8): p. 456-65.
[2] Tam, J., et al., Endocannabinoids in liver disease. Hepatology, 2011. 53(1): p. 346-55.
[3] Matias, I., et al., Regulation, function, and dysregulation of endocannabinoids in models of adipose and beta-pancreatic cells and in obesity and hyperglycemia. J Clin Endocrinol Metab, 2006. 91(8): p. 3171-80.
[4] Osei-Hyiaman, D., et al., Endocannabinoid activation at hepatic CB1 receptors stimulates fatty acid synthesis and contributes to diet-induced obesity. J Clin Invest, 2005. 115(5): p. 1298-305.
[5] Mukhopadhyay, B., et al., Transcriptional regulation of cannabinoid receptor-1 expression in the liver by retinoic acid acting via retinoic acid receptor-gamma. J Biol Chem, 2010. 285(25): p. 19002-11.
[6] Westerbacka, J., et al., Splanchnic balance of freefatty acids, endocannabinoids, and lipids in subjects with nonalcoholic fatty liver disease. Gastroenterology, 2010. 139(6): p. 1961-1971 e1.
[7] Ruby, M.A., et al., Overactive endocannabinoid signaling impairs apolipoprotein E-mediated clearance of triglyceride-rich lipoproteins. Proc Natl Acad Sci U S A, 2008. 105(38): p. 14561-6.
[8] Jourdan, T., et al., CB1 antagonism exerts specific molecular effects on visceral and subcutaneous fat and reverses liver steatosis in diet-induced obese mice. Diabetes, 2010. 59(4): p. 926-34.
[9] Tam, J., et al., Peripheral CB1 cannabinoid receptor blockade improves cardiometabolic risk in mouse models of obesity. J Clin Invest, 2010. 120(8): p. 2953-66.
[10] Son, M.H., et al., Peripherally acting CBI-receptor antagonist: the relative importance of central and peripheral CB1 receptors in adiposity control. Int J Obes (Lond), 2010. 34(3): p. 547-56.
**[11]** Jourdan, T., et al., Antagonism of peripheral hepatic cannabinoid receptor-1 improves liver lipid metabolism in mice: evidence from cultured explants. Hepatology, 2012. 55(3): p. 790-9.
[12] Silvestri, C., et al., Two non-psychoactive cannabinoids reduce intracellular lipid levels and inhibit hepatosteatosis. J Hepatol, 2015. 62(6): p. 1382-90.
[13] Ignatowska-Jankowska, B., M.M. Jankowski, and A.H. Swiergiel, Cannabidiol decreases body weight gain in rats: involvement of CB2 receptors. Neurosci Lett, 2011. 490(1): p. 82-4.
[14] Yang, L., et al., Cannabidiol protects liver from binge alcohol-induced steatosis by mechanisms including inhibition of oxidative stress and increase in autophagy. Free Radic Biol Med, 2014. 68: p. 260-7.
[15] Wargent, E.T., et al., The cannabinoid Delta(9)-tetrahydrocannabivarin (THCV) ameliorates insulin sensitivity in two mouse models of obesity. Nutr Diabetes, 2013. 3: p. e68.
[16] Silvestri C. et al., Two non-psychoactive cannabinoids reduce intracellular lipid levels and inhibit hepatosteatosis. J. Hepatol., 2015, 62(6), 1382-1380.

### GENERAL DESCRIPTION

The present invention aims to explore the therapeutic potential of CBD and/or cannabigerol (CBG) in reversing the accumulation of fat in hepatocytes and determine the downstream molecular pathway(s) involved in this phenomenon; thus providing the means for treating or preventing NAFLD. Without wishing to be bound by the theory, it is believed that phytocannabinoids (alone or in combination) could mitigate hepatic *de novo* lipogenesis or increase hepatic fatty acid β-oxidation to prevent the development of NAFLD as well as reducing hyperglycemia to ameliorate type-2 diabetes (T2D) and treating dyslipidemia.

To that end, the inventors have carried out several experimental paradigms in which they tested whether CBG and/or CBD have the ability to reduce lipid accumulation in hepatocytes. They also tried to decipher the signaling pathway by which phytocannabinoids demonstrate their anti-steatotic effect. Moreover, the inventors tested the therapeutic potential of CBG and/or CBD to treat hepatic steatosis, dyslipidemia, obesity, and hyperglycemia and insulin resistance-induced by high-fat diet.

As known in the art, ***"phytocannabinoids"*** (or generally cannabinoids) are naturally occurring cannabinoids which are produced in plants. Different cannabis species comprise more than 100 different phytocannabinoids. They are divided into subclasses including cannabigerols, cannabichromenes, cannabidiols, tetrahydrocannabinols, cannab-inols, cannabinodiols, and other cannabinoids. Non-limiting examples of phytocannabinoids include cannabigerol (CBG), cannabichromene (CBC), cannabidiol (CBD), tetrahydrocannabinol (THC), cannab-inol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monom-ethyl ether (CBGM), cannabinerolic acid, cannabidiolic acid (CBDA), Cannabinol propyl variant (CBNV), cannabitriol (CBO), tetrahydrocannabinolic acid (THCA), and tetrahy-drocannabivarinic acid (THCVA).

The invention utilizes the effectiveness of CBG or a combination comprising two phytocannabinoids: CBD and CBG to treat NAFLD, dyslipidemia, and T2D or treat or prevent any of the other medical conditions mentioned herein. As noted, medical uses compositions and methods disclosed herein are based on the utilization of CBG alone or CBG in combination with CBD, such that the use of CBG alone or in combination with CBD does not include or involve the presence or use of any other cannabinoid, as defined above. In other words, where the presence or use of other cannabinoids is stated, the composition or use or method will be limited to CBG or CBG in combination with CBD.

Also, as used herein, CBG alone or in combination with CBD, and no other cannabinoids, as disclosed, are considered the active ingredients in the medical uses and compositions mentioned herein. Thus, a composition which comprises at least one cannabinoid consisting of CBG or CBD in combination with CBG, as the active ingredients, or actives, is a composition that may comprise additives and other materials, but will include as active ingredients only CBG or CBD in combination with CBG.

The excluded cannabinoids may be any one or more of cannabichromene (CBC), tetrahydrocannabinol (THC), cannabinol (CBN), cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), cannabinol propyl variant (CBNV), cannabitriol (CBO), tetrahydrocannabinolic acid (THCA) and tetrahy-drocannabivarinic acid (THCVA).

The CBG or CBD used in accordance with the invention are not used in an extract or phytomaterial that may additional cannabinoids.

NAFLD is a major cause of morbidity and mortality in Western societies, encompassing a spectrum of liver conditions ranging from ectopic accumulation of fat in the liver (hepatic steatosis) to NASH, which can be complicated by fibrosis, cirrhosis and HCC. The term *"non-alcoholic fatty liver disease"* thus refers to a medical state wherein a subject suffers from a fatty liver without having a history of alcohol consumption or abuse and in cases where alcohol consumption is not related to the occurrence. Fatty liver is caused by a lipid metabolism disorder or a defect in the process of carrying excessive fat in the liver cells, and is mainly caused by disorders of lipid metabolism in the liver. The fatty liver of subjects suffering from the disease is characterized by an abnormal accumulation of triglyceride in liver cells. If the amount of triglycerides is more than 5% of the liver weight, the liver is classified as a fatty liver. The non-alcoholic fatty liver disease includes manifestations such as NAFLD, NASH, cirrhosis and HCC.

As demonstrated herein, compositions of the present invention provide a greater effectivity in reducing lipids accumulation in hepatocytes when CBG is used or where CBD and CBG are used in a combination, as compared to the effect achieved by CBD when used separately. In addition, the combined effect of CBG and CBD in reducing hypercholesterolinemia was shown to be greater as compared to CBD alone. As such, uses, compositions and methods of the invention, involving CBD and CBG have been found useful in the prevention and treatment of NAFLD, dyslipidemia, and T2D.

Thus, in one of its aspects, the present invention provides cannabigerol (CBG) or a combination thereof with cannabidiol (CBD) for use in the treatment of a non-alcoholic fatty liver disease (NAFLD), dyslipidemia or Type-2 diabetes (T2D) in a subject, wherein the combination or method excludes other cannabinoids.

In some embodiments, the CBG is for use alone (in the absence of CBD) in a treatment of a non-alcoholic fatty liver disease (NAFLD), dyslipidemia or Type-2 diabetes (T2D) in a subject. In other embodiments, a combination with CBD is used, wherein the CBG and CBD are provided separately or together.

In some embodiments, the CBG, alone or in combination with CBD, is for use in a treatment for reducing hepatic triglyceride and cholesterol levels, for reducing hyperglycemia and glucose intolerance, for improving insulin resistance and Type-2 diabetes and/or for reducing blood levels of triglycerides and total cholesterol and LDL-cholesterol levelsin a subject.

The CBD and CBG may be formulated separately or together, as disclosed herein.

The invention further provides a combination of CBD and CBG for use in medicine. In some embodiments, the composition is adapted for treating NAFLD, dyslipidemia, and T2D.

The CBD and CBG may be administered together or separately, such that one of the two may be administrated prior to, after or concomitantly with the administration of the other. In cases where the CBD and CBG are *"administered separately",* one of the two, e.g., may be administered at a time period or in a form that is independent of the administration of the other, e.g., CBG, provided that their separate administrations nevertheless provides a therapeutic effect that is greater than a therapeutic effect achieved by administration of each of the two cannabinoids as two independent monotherapies. Thus, one of CBD and CBG may be administered prior to, after or together with the other, provided that one of the two enhances the effect of the other, resulting in a therapeutic effect that is additive or synergistic. In some embodiments, the synergistic effect is achieved when the ratio between CBD and CBG is between 0.1:1 and 1:0.1, CBD:CBG.

In some embodiments, where CBG is provided in combination with CBD, the combination is a synergistic combination used in the treatment of NAFLD, dyslipidemia or Type-2 diabetes (T2D). In some embodiments, the synergistic combination is for treating NAFLD. In some embodiments, the ratio between CBD and CBG is between 0.1:1 and 1:0.1, CBD : CBG. In some embodiments, the ratio is 1:1.

The two cannabinoids may be administered separately, but at the same time. In such a regimen, each of the cannabinoids is formulated separately and optionally in a different unit dose, for simultaneous or concomitant administration. In some embodiments, the cannabinoids are administered in a unit dose; namely in a physically discrete unit which is packaged individually and is suitable for administration to the subject (human or non-human). Each unit dose can contain an effective amount- a prescribed quantity of CBD and/or CBG sufficient to produce a therapeutic effect. Unit dose forms can be administered in portions or multiples thereof. The invention further contemplates the use of CBD in combination with CBG in a treatment of preventing or treating NAFLD and/or dyslipidemia and/or T2D in a subject.

Embodiments of the current invention can also be utilized to form a medicament or a pharmaceutical composition, which contains CBG or a combination of CBG and CBD and further optionally at least one carrier, excipient or an additive. Where a combination is used, one can utilize the additive or synergistic effect of a combination of CBD and CBG and use it as an effective method of treating NAFLD or any of the other medical conditions disclosed herein. Thus, in another one of its aspects, the invention discloses the use of CBG or a combination of CBD and CBG for the preparation of a medicament for preventing or treating NAFLD and/or dyslipidemia and/or T2D in a subject.

Also provided is a medicament or a pharmaceutical composition comprising CBG or, separately or together, CBD and CBG, for simultaneous or sequential administration.

*A **"medicament"*** or a ***"pharmaceutical composition"*** of the invention comprises a therapeutically effective amount of CBG or a combination of CBD and CBG, separately or together, optionally with suitable additives such as diluents, preservatives, solubilizers, emulsifiers, adjuvant and/or carriers. The compositions may be liquids or lyophilized or otherwise dried formulations and include diluents of various buffer content (e.g., Tris-HCl, acetate, phosphate), pH and ionic strength, additives such as albumin or gelatin to prevent absorption to surfaces, detergents (e.g., Tween 20, Tween 80, Pluronic F68, bile acid salts), solubilizing agents (e.g., glycerol, polyethylene glycerol), anti-oxidants (e.g., ascorbic acid, sodium metabisulfite), preservatives (e.g., Thimerosal, benzyl alcohol, parabens), and others.

Compositions suitable for oral administration can comprise of (a) liquid solutions, such as an effective amount of CBG or the CBD/CBG dissolved in diluents, such as water, saline, or orange juice; (b) capsules, sachets, tablets, lozenges, and troches, each containing a predetermined amount of the active ingredients, as solids or granules; (c) powders; (d) suspensions in an appropriate liquid; and (e) suitable emulsions or self-emulsifying formulations. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers. Tablet forms can include one or more of lactose, sucrose, mannitol, corn starch, potato starch, alginic acid, microcrystalline cellulose, acacia, gelatin, guar gum, colloidal silicon dioxide, croscarmellose sodium talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredients, such carriers as are known in the art.

Compositions suitable for parenteral administration include sterile nanoemulsions, aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that include suspending agents, solubilizers, thickening agents, stabilizers, and preservatives.

The CBG or a combination of CBD and CBG can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants. Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid.

The CBG or a combination of CBD and CBG may be also made into injectable formulations. The requirements for effective pharmaceutical carriers for injectable compositions are well known to those of ordinary skill in the art. See Pharmaceutics and Pharmacy Practice, J.B. Lippincott Co., Philadelphia, Pa., Banker and Chalmers, eds., pages 238-250 (1982), and ASHP Handbook on Injectable Drugs, Toissel, 4th ed., pages 622-630 (1986).

It is described that, the composition is a saline composition comprising also at least one alcohol such as ethanol.

It is described that, the composition comprises cremophor.

It is described that, the composition of the invention is formed in an ethanol:cremophor:saline formulation, optionally comprising a ratio of 1:1:18 ethanol: cremophor: saline.

Each of CBD and CBG, independently, as well as a combination of the two, may be in a form suitable for oral, parenteral, subcutaneous, intravenous, sublingual, intramuscular or interperitoneal administration. In some embodiments, the combination of CBD and CBG is suitable for oral administration. In other embodiments, the combination is suitable for sc, iv or ip administration.

It is described that, one of CBD and CBG is administered orally and the other of CBD and CBG is administered in a different mode of administration.

As exemplified herein, compositions of the invention comprising, separately or together, a combination of CBD and CBG enhance the effectiveness in the treatment of NAFLD and/or dyslipidemia and/or T2D or other conditions as compared to the use of each one of the cannabinoids separately. Therefore, such compositions may be used in methods of treatment or prophylaxis of NAFLD and/or dyslipidemia and/or T2D.

There can be various ratios between CBD and CBG in any of the compositions or medicaments described hereinabove. Thus, in some embodiments, the composition may comprise CBD and CBG at a ratio of between 0.1:1 to a ratio of 1:0.1, CBD:CBG. In some embodiments, the ratio between CBD and CBG is 1:1, 1:1.5, 1:2, 1:2.5, 1:3, 1:3.5, 1:4, 1:4.5, 1:5, 5:1, 4.5:1, 4:1, 3.5:1, 3:1, 2.5:1 or 2:1. In some embodiments, the ratio is 1:1. In some other embodiments, the ratio between CBD and CBG is 1:2.5.

In some embodiments, the composition is used for reducing hepatic triglyceride and cholesterol levels, for reducing hyperglycemia and glucose intolerance, for improving insulin resistance and Type-2 diabetes and/or for reducing blood levels of triglycerides and total cholesterol and LDL-cholesterol levelsin a subject.

Thus, in another one of its aspects, the present invention discloses a method of increasing the effectiveness of CBD or CBG in the treatment of NAFLD and/or dyslipidemia and/or T2D, wherein the method comprising administering to a subject in need thereof CBD prior to, after or concomitantly with CBG.

The invention also provides a method of treating or preventing NAFLD and/or dyslipidemia and/or T2D in a subject, the method comprising administering to the subject CBG or a combination of CBD and CBG, separately or together.

In some embodiments, the CBD and CBG are administered in combination.

In some embodiments, the combination comprises an effective amount of one of CBD and CBG sufficient to enhance a therapeutic effect exerted by the other of CBD and CBG.

In some embodiments, the effect exerted by administering a combination of CBD and CBG is a synergetic effect.

In another aspect, the invention provides a method for inducing anti-steatotic effect (e.g., by changing the expression profile of genes and transcription factors) and reducing dyslipidemia, hyperglycemia, glucose tolerance and insulin resistance in a subject, the method comprising administering to the subject CBD and CBG.

In another aspect, there is provided a method of reversing accumulation of fat in hepatocyte cells, and reducing dyslipidemia, hyperglycemia, glucose tolerance and insulin resistance, the method comprising administering to a subject in need thereof CBD and CBG.

The invention further provides a method of preventing or treating a non-alcoholic fatty liver disease (NAFLD), dyslipidemia or Type-2 diabetes (T2D) in a subject, the method comprising administering to the subject CBG alone or in combination with CBD, wherein when CBD and CBG are administered in combination, the CBD and CBG are administered together or separately, wherein the method excludes administration of another cannabinoid.

In some embodiments of methods of the invention, the CBD and CBG are administered in combination.

In some embodiments, the combination comprises an effective amount of CBD or CBG, said effective amount being sufficient to enhance a therapeutic effect exerted by the other of CBD and CBG. In some embodiments, the effect exerted by administering a combination of CBD and CBG is a synergetic effect.

In some embodiments, use of the invention are for inducing anti-steatotic effect in a subject, the method comprising administering to the subject CBG alone or a combination of CBD and CBG; for reversing accumulation of fat in hepatocyte cells, the method comprising administering to a subject in need thereof CBG alone or a combination of CBD and CBG; for reducing hepatic triglyceride and cholesterol levels, for reducing hyperglycemia and glucose intolerance, for improving insulin resistance and Type-2 diabetes and/or for reducing blood levels of triglycerides and total cholesterol and LDL-cholesterol levels, the use comprising administering to a subject in need thereof CBG alone or a combination of CBD and CBG.

The invention also provides a use in the treatment of a subject, the use comprising administering to the subject CBG as the sole active ingredient, for reducing hepatic triglyceride and cholesterol levels, reducing hyperglycemia and glucose intolerance, improving insulin resistance and Type-2 diabetes, reducing blood levels of triglycerides and total cholesterol and LDL-cholesterol levels.

Also provided is CBG in combination with CBD, as the sole active ingredients, for use in the treatment of a subject for reducing hepatic triglyceride and cholesterol levels, reducing hyperglycemia and glucose intolerance, improving insulin resistance and Type-2 diabetes, reducing blood levels of triglycerides and total cholesterol and LDL-cholesterol levels.

Also provided is a compound for use in the treatment of NAFLD and/or dyslipidemia and/or T2D in a subject, the compound being selected from CBD and CBG.

In some embodiments, the compound is a combination of CBD and CBG. In some embodiments, the CBD is administered prior to, after or concomitantly with CBG. In some embodiments, each of the CBD and CBG are separately administered. In some embodiments, each of the CBD and CBG are administered in a unit dose.

Thus, the invention provides the following aspects and embodiments of the invention:
Cannabigerol (CBG) or a combination thereof with cannabidiol (CBD) for use in treating a non-alcoholic fatty liver disease (NAFLD), dyslipidemia or Type-2 diabetes (T2D) in a subject, wherein the combination excludes other cannabinoids.

CBG or combination with CBD for use alone in treating a non-alcoholic fatty liver disease (NAFLD), dyslipidemia or Type-2 diabetes (T2D) in a subject.

CBG or combination with CBD, for use in combination with CBD, wherein the CBG and CBD are provided separately or together.

CBG provided alone or in combination with CBD, for use in reducing hepatic triglyceride and cholesterol levels, for reducing hyperglycemia and glucose intolerance, for improving insulin resistance and Type-2 diabetes and/or for reducing blood levels of triglycerides and total cholesterol and LDL-cholesterol levels.

CBG provided in combination, wherein the CBD and CBG are formulated separately.

CBG provided in combination, wherein the CBD and CBG are formulated together.

CBG alone or in combination, when formulated as a pharmaceutical composition.

CBG in combination with CBD, wherein the combination is a synergistic combination in the treatment of NAFLD, dyslipidemia or Type-2 diabetes (T2D).

CBG in combination with CBD, the combination being a synergistic combination in the treatment of NAFLD.

The combination wherein the ratio between CBD and CBG is between 0.1:1 and 1:0.1, CBD : CBG. The ratio may be 1:1.

Also provided is a pharmaceutical composition comprising CBG, alone or in combination with CBD, for use in preventing or treating a non-alcoholic fatty liver disease, dyslipidemia or Type-2 diabetes (T2D) in a subject, wherein the composition is free of other cannabinoids.

A composition comprises at least one cannabinoid consisting CBG or CBG in combination with CBD.

A composition comprises CBD and CBG, wherein the CBD and CBG are separately contained.

A composition comprises CBD and CBG, wherein the CBD is adapted for administration prior to, after or concomitantly with the CBG.

A composition comprises CBD and CBG, wherein CBD and CBG are in different or identical amount.

A composition comprises CBD and CBG, wherein the ratio between CBD and CBG is between 0.1:1 and 1:0.1, CBD : CBG. The ratio may be 1:1 or 1:2.5.

A composition comprises CBG and free of CBD.

A composition for use in a method of treatment comprising administration of CBD.

A composition for use in reducing hepatic triglyceride and cholesterol levels, for reducing hyperglycemia and glucose intolerance, for improving insulin resistance and Type-2 diabetes and/or for reducing blood levels of triglycerides and total cholesterol and LDL-cholesterol levels.

A unit dose comprising CBG, CBD or a combination thereof, for use in preventing or treating a non-alcoholic fatty liver disease, dyslipidemia or Type-2 diabetes (T2D), wherein the unit dose excludes another cannabinoid.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
Fig. 1 depicts the effect of CBD in ameliorating Oleate:Palmitate-induced lipid accumulation in hepatocytes.
Fig. 2 presents the effect of CBG in ameliorating Oleate:Palmitate-induced lipid accumulation in hepatocytes.
Fig. 3 depicts the Effect of CBD+CBG in ameliorating Oleate:Palmitate-induced lipid accumulation in hepatocytes.
Fig. 4 depicts the effect of CBD+CBG on gene expression profile in hepatocytes exposed to Oleate:Palmitate.
Figs. 5A-C depict the isolated and combined effects of CBD and CBG on hepatic triglyceride and cholesterol contents in high-fat diet-induced obese mice
Figs. 6A-C depict the isolated and combined effects of CBD and CBG on glucose homeostasis in high-fat diet-induced obese mice
Figs. 7A-B depict the isolated and combined effects of CBD and CBG on insulin sensitivity in high-fat diet-induced obese mice.
Figs. 8A-C depict the isolated and combined effects of CBD and CBG on circulating triglycerides and cholesterol in high-fat diet-induced obese mice.
Figs. 9A-C depict the isolated and combined effects of CBD and CBG on body weight and mass in high-fat diet-induced obese mice.

### DETAILED DESCRIPTION OF EMBODIMENTS

The current invention is based on the therapeutic potential of CBD and/or CBG in reversing the accumulation of fat in hepatocytes, and determine the downstream molecular pathway(s) involved in this phenomenon. It also demonstrates the therapeutic potential of CBD and/or CBG in ameliorating dyslipidemia, body mass, hyperglycemia, glucose tolerance and insulin resistance. To demonstrate the efficacy of CBD and/or CBG, we carried out several experimental paradigms in which we tested whether CBG and/or CBD have the ability to reduce lipid accumulation in hepatocytes. We also tried to decipher the signaling pathway by which phytocannabinoids demonstrate their antisteatotic effect. We then demonstrated the effectiveness of CBD and/or CBG in reducing hepatic steatosis, hypercholesterolinemia, hypertriglyceridemia, obesity, hyperglycemia, glucose homeostasis and insulin resistance in high-fat diet-induced obese mice treated chronically with CBD and/or CBG for 28 days.

### Research progress and results:

### In Vitro Analysis:

To test whether CBG and/or CBD have the ability to reduce lipid accumulation in hepatocytes, we have utilized an *in vitro* model of fat accumulation in hepatocytes. Briefly, human hepatoma HepG2 cells were maintained at 37°C in 5% CO₂ in DMEM medium supplemented with 10% FCS, 2 mM l-glutamine, 1 mM sodium pyruvate, 100 U/mL penicillin and 100 mg/mL streptomycin. Free fatty acids (2:1, molar ratio, oleic and palmitic acids) were mixed with bovine serum albumin (BSA). Cells were incubated with fatty acid-BSA complex in FCS-free culture medium at 0.3 mM final concentration of fatty acid and 1% of BSA. Control cell cultures were incubated with medium containing the vehicle. Compounds were tested in a range of concentrations (as detailed in each figure) in the presence/absence of fatty acids. The synergistic effect between two compounds was also tested once validating their individual effect. After 24 hr of incubation with the compounds, the cells were washed, incubated with 1 µg/mL mixture of Nile-Red/Hoechst solution for 15 mins at 37°C protected from light. Fluorescence was measured by the Cytation-3 plate reader at ex:488nm/em:550 for Nile-Red and Hoechst, respectively. Results were normalized to total protein levels of each well, and presented as a change in the accumulation of lipids in comparison with Vehicle-treated group.

### Findings:

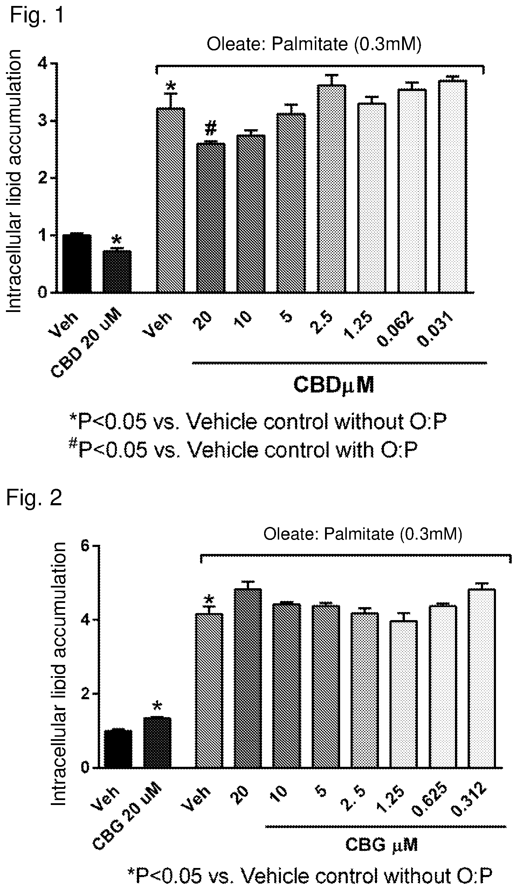

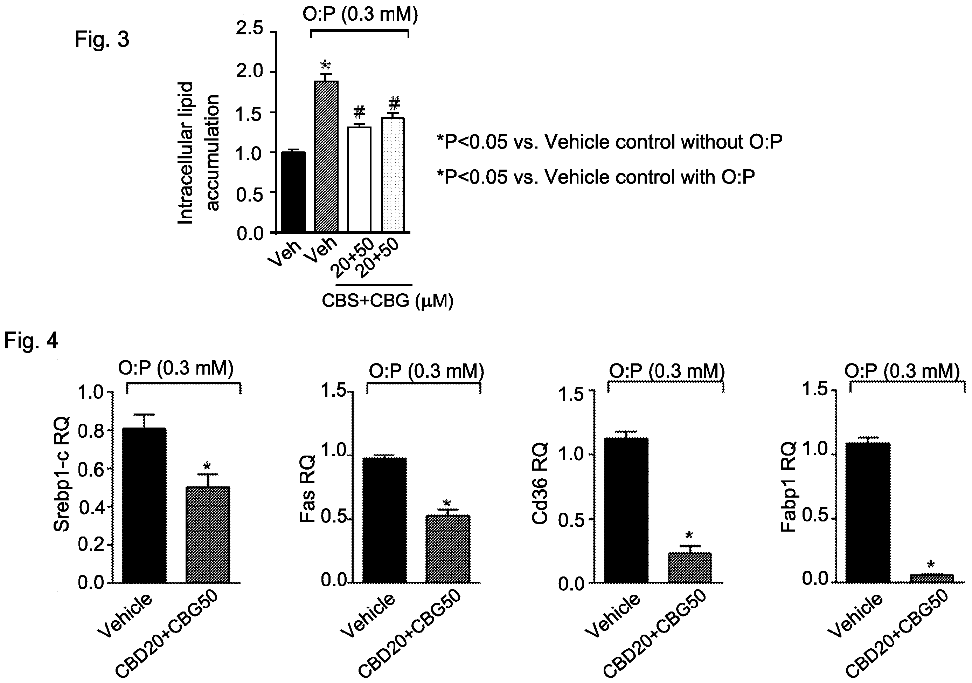

Our findings show that CBD in a concentration of 20 µM was able to significantly reduce lipid accumulation in hepatocytes (Fig. 1), whereas CBG was not as effective as CBD in any dose tested (Fig. 2). Nevertheless, the combined effect of both CBD and CBG seemed to have a synergistic effect, as both compounds (in concentrations of 20 + 50 µM, respectively; and 20 + 20 µM, respectively) were able to significantly prevent the accumulation of lipids in hepatocytes (Fig. 3).

To further decipher the signaling pathway by which phytocannabinoids demonstrate their antisteatotic effect, we performed gene array analysis to measure the expression levels of many enzymes and transcription factors, following exposure to free fatty acids (2: 1, molar ratio, oleic and palmitic acids) in the presence/absence of CBD + CBG at concentrations of 20 and 50 µM, respectively (as found in *Aim I*)*.* Total mRNA from HepG2 cells was extracted using Bio-Tri RNA lysis buffer (Bio-Lab, Israel) followed by DNase I treatment (Thermo Scientific, IL, USA), and reverse transcribed using the Iscript cDNA kit (Bio-Rad, CA). Real-time PCR was performed using the iTaq Universal SYBR Green Supermix (Bio-Rad, CA) and the CFX connect ST system (Bio-Rad, CA).

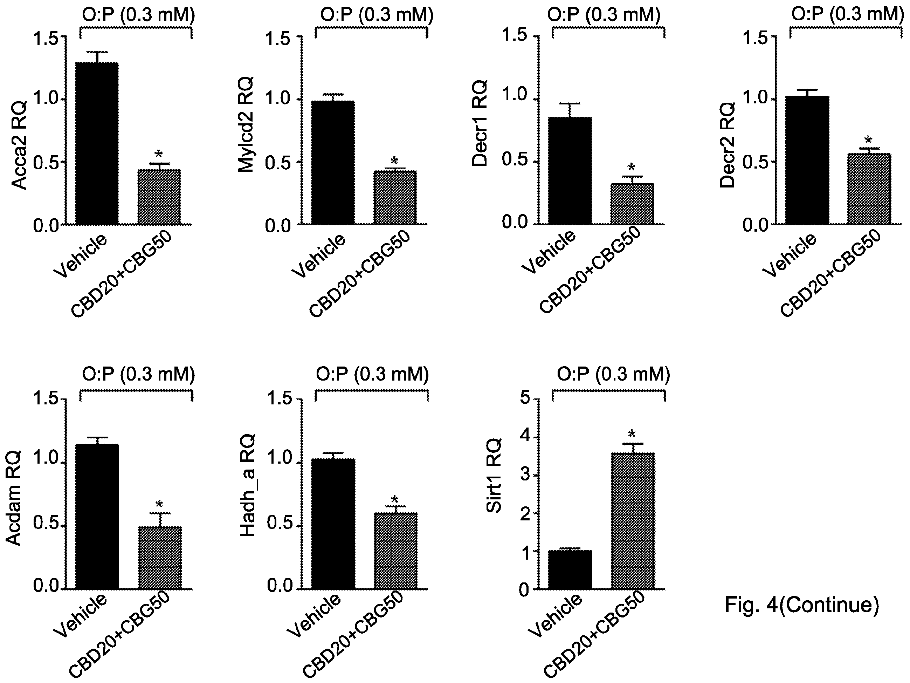

Our findings show that a mixture of CBD (20 µM) and CBG (50 µM) was able to change the expression profile of many genes and transcription factors involved in *de novo* fatty acid synthesis (lipogenesis); and mitochondrial fatty acid β-oxidation. As can be seen in Fig. 4, the expression of most of the genes responsible for *de novo* lipogenesis *(Srebplc, Fas, Acca2, Mylcd2, Decr1, Decr2, Acdam, Hadh),* and transport of fatty acids *(Cd36, Fabpl)* was significantly reduced by CBD+CBG, whereas the expression of *Sirt1* as a master regulator of fatty acid β-oxidation was upregulated.

### Summary of in vitro findings:

1. CBD has the potential to inhibit lipids accumulation in HepG2 cells. These results were verified by running three independent studies.
2. CBG seems not to affect lipid accumulation in HepG2 cells. These results were verified by running three independent studies.
3. The inhibitory effect of CBD on lipid accumulation was significantly enhanced in combination with CBG. These results were verified by running three independent studies.
4. The combined effect of CBD and CBG seems to induce positive changes in the expression of genes responsible for fatty acid accumulation, transport and utilization.

### In Vivo Analysis:

Next, the inventors tested whether the isolated and combined effect of phytocannabinoids has the ability to reverse fatty liver (hepatic steatosis) and improve metabolism in high-fat diet-induced obese mouse. Briefly, six weeks old, male C57Bl/6J mice were fed *ad libitum* with a high-fat diet (HFD; D12492; 60% of calories from fat, 20% from protein, and 20% from carbohydrates; Research Diets) or a mouse standard diet (STD; NIH no. 31 rodent diet) for 14 weeks. Then, the mice were treated for 28 days with CBD (10 mg/kg/day, ip), CBG (25 mg/kg/day, ip), CBD+CBG (10 mg/kg/day+25mg/kg/day, ip respectively) or vehicle (EtOH:cremophor:saline; 1:1:18). The mouse groups were labeled as defined here: mice on a STD treated with vehicle (STD-Veh), mice on a HFD treated with vehicle (HFD-Veh), mice on a HFD treated with CBD (HFD-CBD), mice on a HFD treated with CBG (HFD-CBG), and mice on a HFD treated with CBD+CBG (HFD-CBD+CBG).

Metabolic analyses were carried out as detailed herein:
Hepatic steatosis (Fatty liver) was measured by (i) quantifying the amount of triglycerides and cholesterol in the liver by using a specific lipid extraction method followed by colorimetric chemical analyses using the Cobas C111 Chemistry Analyzer (Roche, Germany), and (ii) evaluating the reversal of fatty liver by histology. Briefly, paraffin-embedded liver sections (5 µM) were be used to evaluate hepatic morphology (using H & E staining).

Glucose and insulin homeostasis was assessed by (i) measuring fasting blood glucose (Elite glucometer, Bayer, Germany) and serum insulin (Ultra-Sensitive Mouse Insulin ELISA kit, EMD Millipore, MA) levels, (ii) evaluating Homeostatic Model Assessment for Insulin Resistance (HOMA-IR), and (iii) performing intraperitoneal glucose tolerance test.

Lipid balance was assessed by evaluating the levels of low-density lipoprotein (LDL)-cholesterol, total cholesterol and triglycerides by using Cobas C111 Chemistry Analyzer (Roche, Germany).

Antiobesity effect was assessed by measuring body weight. Total fat and lean masses were determined at the end of the treatment by EchoMRI-100 (Echo Medical Systems LLC, Houston TX).

### Findings:

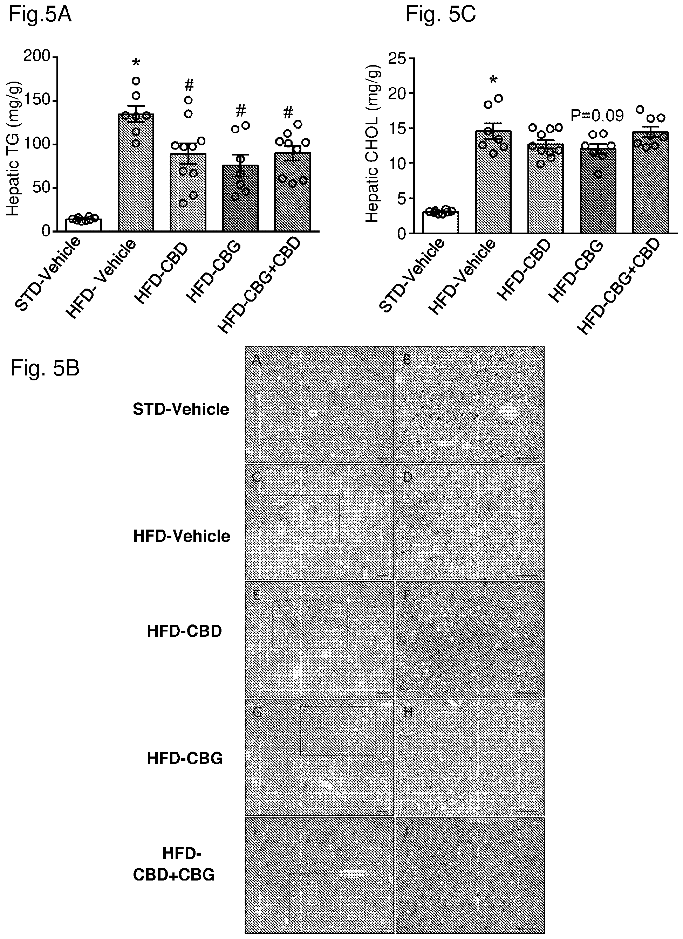

The hepatic and metabolic effects of CBD and/or CBG were examined in high-fat diet-induced obese mice. Male C57BL/6 mice fed a high-fat diet (HFD) for 14 weeks became obese and were then started on daily ip injections of vehicle, or drugs (as indicated above) for an additional 28 days. Age- and sex-matched mice on STD and treated with vehicle served as controls. The HFD-induced hepatic steatosis (fatty liver), as reflected by the elevated triglyceride levels in the liver as well as the increased fat vacuoles and ballooning in hepatocytes, was significantly attenuated by CBD, CBG, and CBD+CBG **(Fig. 5A, B).** A trend toward reduction in liver cholesterol levels was only recorded in the CBG-treated group **(****Fig. 5C****).**

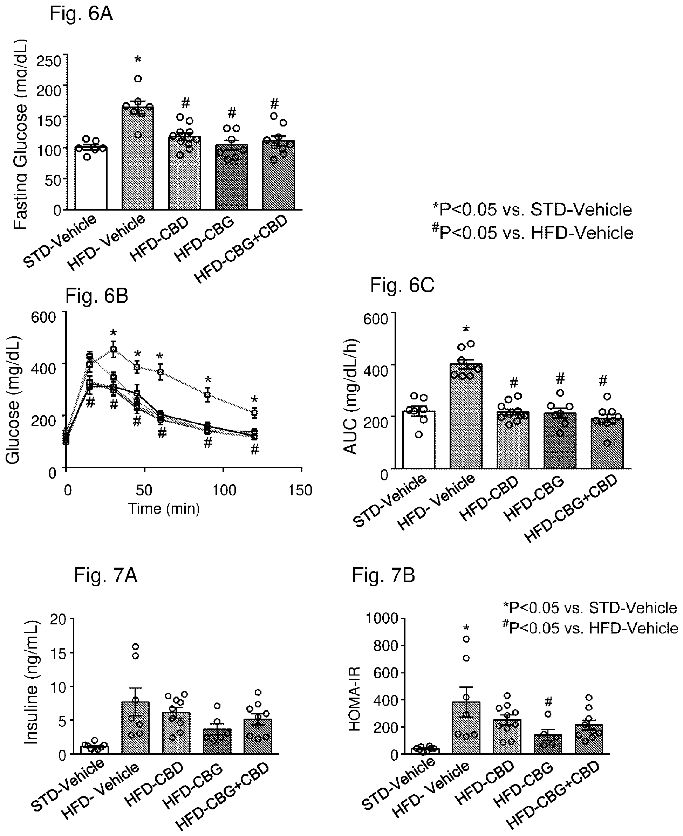

Glucose homeostasis was assessed by measuring fasting blood glucose levels as well as assessing the ability of the animals to overcome a bolus of glucose over time. Specifically, the HFD-induced hyperglycemia was significantly normalized by CBD, CBG, and CBD+CBG to the level seen in STD-fed animals **(****Fig. 6A****).** This reduction in fasting blood glucose levels were translated into a significant improvement in glucose tolerance in HFD-induced obese mice treated with CBD, CBG, and CBD+CBG **(**Fig. 6B, C).

Next, insulin sensitivity was measured by evaluating serum insulin levels and HOMA-IR. Whereas CBD was found to be ineffective in reducing hyperinsulinemia, a trend toward reduction in insulin levels was found with CBG treatment **(****Fig. 7A****),** suggesting improved insulin homeostasis. Indeed, normalization of HOMA-IR, a marker for β-cell function and insulin resistance, was only found in the HFD-fed mice treated chronically with CBG **(****Fig. 7B****).**

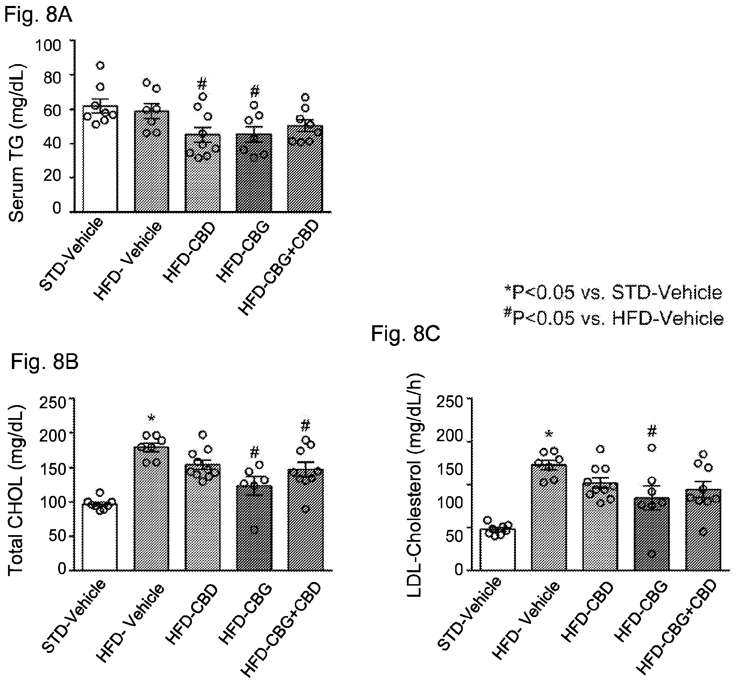

The effect of the phytocannabinoids on dyslipidemia was further assessed by measuring circulating levels of cholesterol and triglycerides. Reduction in serum triglycerides were found with CBG and CBD **(****Fig. 8A****).** Total circulating cholesterol levels were significantly reduced by CBG and CBG+CBD **(****Fig. 8B****).** The reduction in total cholesterol was mainly attributed to a significant reduction in LDL-Cholesterol levels recorded in the CBG-treated animals **(****Fig. 8C****).**

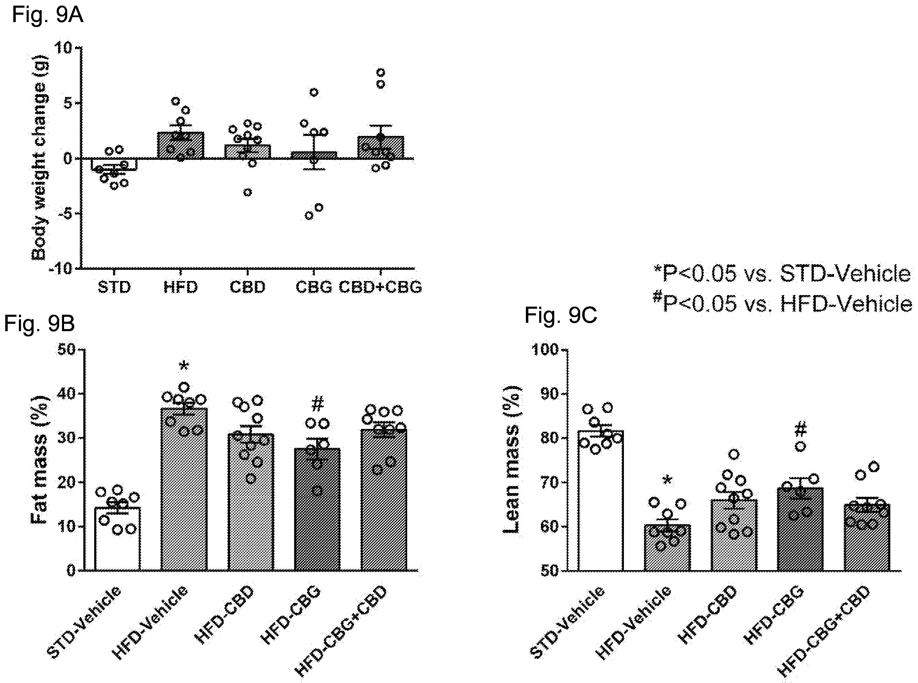

All the above-mentioned findings were not related to body weight change **(****Fig. 9A****).** Yet, significant reduction in fat mass and increased lean mass were found in the CBG-treated mice **(**Fig. 9B, C).

### Summary of in vivo findings:

1. CBG has the ability to:
   a. Reduce hepatic triglyceride and cholesterol levels
   b. Reduce hyperglycemia and glucose intolerance
   c. Improve insulin resistance and T2D
   d. Reduce blood levels of triglycerides, total cholesterol and LDL-Cholesterol
   e. Reduce fat mass
   f. Increase lean mass
2. CBD has the ability to:
   a. Reduce hepatic triglyceride levels
   b. Reduce hyperglycemia and glucose intolerance
   c. Reduce blood levels of triglycerides
3. CBD and CBG have the ability to:
   a. Reduce hepatic triglyceride levels
   b. Reduce hyperglycemia and glucose intolerance
   c. Reduce blood levels of total cholesterol

## Claims

1. Cannabigerol (CBG) alone or a combination with cannabidiol (CBD) for use in the treatment of a non-alcoholic fatty liver disease (NAFLD), or the treatment or prevention of dyslipidemia, or the treatment or prevention of Type-2 diabetes (T2D), or the reduction of hepatic triglyceride and cholesterol levels, or the treatment or reduction of hyperglycemia and glucose intolerance, or the treatment or improvement of insulin resistance and Type-2 diabetes, or the reduction of blood levels of triglycerides and total cholesterol and LDL-cholesterol levels in a subject, wherein said use excludes other cannabinoids.

2. CBG for use according to claim 1 in combination with CBD, wherein the CBG and CBD are provided separately or together.

3. CBG for use according to claim 1 or 2, wherein the CBD and CBG are formulated separately.

4. CBG for use according to claim 1 or 2, wherein the CBD and CBG are formulated together.

5. CBG for use according to claim 1 or 2, formulated as a pharmaceutical composition.

6. A pharmaceutical composition comprising CBG, alone or in combination with CBD, for use according to claim 1.

7. The composition for use according to claim 6, comprising at least one cannabinoid consisting of CBG or CBG in combination with CBD.

8. The composition for use according to claim 6, comprising CBD and CBG, wherein the CBD is adapted for administration prior to, after or concomitantly with the CBG.

9. The composition for use according to any one of claims 6 to 8, comprising CBD and CBG, wherein CBD and CBG are in different or identical amount.

10. The composition for use according to claim 6, comprising CBD and CBG, wherein the ratio between CBD and CBG is between 0.1:1 and 1:0.1, CBD: CBG.

11. The composition for use according to claim 10, wherein the ratio between CBD and CBG is 1:1.

12. The composition for use according to claim 6, comprising CBG and being free of CBD.

13. The composition for use according to claim 12, in a treatment according to claim 1 comprising administration of CBD.

14. The composition for use according to any one of claims 6 to 13, for the reduction of hepatic triglyceride and cholesterol levels, the treatment or reduction of hyperglycemia and glucose intolerance, the treatment or reduction of insulin resistance and Type-2 diabetes and/or the reduction of blood levels of triglycerides and total cholesterol and LDL-cholesterol levels.

15. The composition for use according to the claims 6 - 14 prepared for oral administration.

16. The composition for use according to the claims 6 - 14 prepared for intravenous administration.

## Patentansprüche

1. Cannabigerol (CBG) allein oder eine Kombination mit Cannabidiol (CBD) zur Verwendung bei der Behandlung einer nicht-alkoholischen Fettlebererkrankung (NAFLD) oder der Behandlung oder Prävention von Dyslipidämie oder der Behandlung oder Prävention von Typ-2-Diabetes (T2D) oder der Reduzierung der Leber-Triglycerid- und Cholesterinspiegel oder der Behandlung oder Reduzierung von Hyperglykämie und Glukoseintoleranz oder der Behandlung oder Verbesserung von Insulinresistenz und Typ-2-Diabetes oder der Reduzierung der Blutspiegel von Triglyceriden und Gesamtcholesterin- und LDL-Cholesterinspiegel bei einem Patienten, wobei die Verwendung andere Cannabinoide ausschließt.

2. CBG zur Verwendung gemäß Anspruch 1 in Kombination mit CBD, wobei CBG und CBD separat oder zusammen bereitgestellt sind.

3. CBG zur Verwendung gemäß Anspruch 1 oder 2, wobei CBD und CBG separat formuliert sind.

4. CBG zur Verwendung gemäß Anspruch 1 oder 2, wobei CBD und CBG zusammen formuliert sind.

5. CBG zur Verwendung gemäß Anspruch 1 oder 2, formuliert als pharmazeutische Zusammensetzung.

6. Pharmazeutische Zusammensetzung, die CBG allein oder in Kombination mit CBD umfasst, zur Verwendung gemäß Anspruch 1.

7. Zusammensetzung zur Verwendung gemäß Anspruch 6, die zumindest ein Cannabinoid umfasst, das aus CBG oder aus CBG in Kombination mit CBD besteht.

8. Zusammensetzung zur Verwendung gemäß Anspruch 6, die CBD und CBG umfasst, wobei das CBD zur Verabreichung vor, nach oder zugleich mit dem CBG angepasst ist.

9. Zusammensetzung zur Verwendung gemäß einem der Ansprüche 6 bis 8, die CBD und CBG umfasst, wobei CBD und CBG in der Menge unterschiedlich oder identisch sind.

10. Zusammensetzung zur Verwendung gemäß Anspruch 6, die CBD und CBG umfasst, wobei das Verhältnis zwischen CBD und CBG zwischen 0,1:1 und 1:0,1, CBD: CBG beträgt.

11. Zusammensetzung zur Verwendung gemäß Anspruch 10, wobei das Verhältnis zwischen CBD und CBG 1:1 beträgt.

12. Zusammensetzung zur Verwendung gemäß Anspruch 6, die CBG umfasst und von CBD frei ist.

13. Zusammensetzung zur Verwendung gemäß Anspruch 12 in einer Behandlung gemäß Anspruch 1, die die Verabreichung von CBD umfasst.

14. Zusammensetzung zur Verwendung gemäß einem der Ansprüche 6 bis 13 für die Reduzierung der Leber-Triglycerid- und Cholesterinspiegel, die Behandlung oder Reduzierung von Hyperglykämie und Glukoseintoleranz, die Behandlung oder Reduzierung von Insulinresistenz und Typ-2-Diabetes und/oder die Reduzierung der Blutspiegel von Triglyceriden und Gesamtcholesterin- und LDL-Cholesterinspiegel.

15. Zusammensetzung zur Verwendung gemäß den Ansprüchen 6 bis 14, die zur oralen Verabreichung präpariert ist.

16. Zusammensetzung zur Verwendung gemäß den Ansprüchen 6 bis 14, die zur intravenösen Verabreichung präpariert ist.

## Revendications

1. Le cannabigérol (CBG), seul ou en association avec le cannabidiol (CBD), destiné au traitement de la stéatose hépatique non alcoolique (NAFLD), ou au traitement ou à la prévention de la dyslipidémie, ou au traitement ou à la prévention du diabète de type 2 (DT2), ou à la réduction des taux hépatiques de triglycérides et de cholestérol, ou au traitement ou à la réduction de l'hyperglycémie et de l'intolérance au glucose, ou au traitement ou à l'amélioration de la résistance à l'insuline et du diabète de type 2, ou à la réduction des taux sanguins de triglycérides et du cholestérol total et des taux de cholestérol LDL chez un sujet, ladite utilisation excluant d'autres cannabinoïdes.

2. CBG destiné à être utilisé selon la revendication 1 en association avec du CBD, le CBG et le CBD étant fournis séparément ou ensemble.

3. CBG destiné à être utilisé selon la revendication 1 ou 2, dans lequel le CBD et le CBG sont formulés séparément.

4. CBG destiné à être utilisé selon la revendication 1 ou 2, dans lequel le CBD et le CBG sont formulés ensemble.

5. CBG destiné à être utilisé selon la revendication 1 ou 2, formulé sous forme de composition pharmaceutique.

6. Composition pharmaceutique comprenant du CBG, seul ou en association avec du CBD, destinée à l'utilisation selon la revendication 1.

7. Composition destinée à être utilisée selon la revendication 6, comprenant au moins un cannabinoïde constitué de CBG ou de CBG en association avec du CBD.

8. Composition destinée à être utilisée selon la revendication 6, comprenant du CBD et du CBG, dans laquelle le CBD est adapté pour être administré avant, après ou simultanément au CBG.

9. Composition destinée à être utilisée selon l'une quelconque des revendications 6 à 8, comprenant du CBD et du CBG, dans laquelle le CBD et le CBG sont présents en quantités différentes ou identiques.

10. Composition destinée à être utilisée selon la revendication 6, comprenant du CBD et du CBG, dans laquelle le rapport entre le CBD et le CBG est compris entre 0,1:1 et 1:0,1, CBD:CBG.

11. Composition destinée à être utilisée selon la revendication 10, dans laquelle le rapport entre le CBD et le CBG est de 1:1.

12. Composition destinée à être utilisée selon la revendication 6, comprenant du CBG et exempte de CBD.

13. Composition destinée à être utilisée selon la revendication 12, dans le cadre d'un traitement selon la revendication 1 comprenant l'administration de CBD.

14. Composition destinée à être utilisée selon l'une quelconque des revendications 6 à 13, pour la réduction des taux hépatiques de triglycérides et de cholestérol, le traitement ou la réduction de l'hyperglycémie et de l'intolérance au glucose, le traitement ou la réduction de la résistance à l'insuline et du diabète de type 2 et/ou la réduction des taux sanguins de triglycérides, de cholestérol total et de cholestérol LDL.

15. Composition destinée à être utilisée selon les revendications 6 à 14, préparée pour une administration par voie orale.

16. Composition destinée à être utilisée selon les revendications 6 à 14, préparée pour une administration intraveineuse.
